# EUROPEAN PATENT SPECIFICATION

(11) **EP 0 976 395 B1**
(45) Date of publication and mention of the grant of the patent: **19.10.2005**
(21) Application number: 98401956.2
(22) Date of filing: 30.07.1998
(51) Int. Cl.: A61K 9/46, A61K 31/155

(54) **Tablet for extended release of a drug in the stomach**
Tablette mit verlängerter Wirkstoffabgabe im Magen
Comprimé à libération prolongée d'un médicament dans l'estomac

(43) Date of publication of application: 02.02.2000
(73) Proprietor: Merck Sante, 69008 Lyon (FR)
(72) Inventor: Bonhomme, Yves, 69260 Charbonnieres les Bains (FR); Nicholson, Geoffroy, Aylesbury, Buckinghamshire HP 21 9 UT (GB)
(74) Representative: Bernasconi, Jean Raymond

(56) References cited:
- EP-A- 0 235 718
- EP-A- 0 283 369
- EP-A- 0 455 475

## Description

Chronic illness is often treated with medication that involves multiple daily doses of a particular therapeutic entity. Patient compliance and therefore efficacy of therapy, may be improved by use of an extended release formulation, for example a hydrophilic matrix tablet that allows once daily dosing of medication.

The rational design and evaluation of effective extended release delivery systems needs to take into account several parameters:
(1) the delivery system,
(2) physicochemical properties of the drug, and
(3) physiological considerations.

Each of these is inter-related to the other two and affects the rate at which the drug is absorbed throughout the GI tract and its ultimate bioavailability and pharamcokinetic profile. These three parameters are considered below.
(1) The range of delivery systems available for the controlled/extended release of drugs is huge. In summary, the nature of the delivery system will be dictated by the properties and dose of the drug, desired release profile and physiological factors. For example, it would prove challenging to develop an extended release system for a high dose, water soluble drug with a narrow absorption window limited to either the stomach and/or the upper intestine as defined by its pKa or site of active transport mechanism.
(2) The physicochemical properties of a drug will affect its absorption through the GI tract. Many drugs are, or are the salts of weak bases or weak acids, and as such demonstrate pH dependent solubility. An extension of this theory is the pH partition hypothesis which asserts that the passage rate of a drug through a membrane is dependent on the environment pH and pKa of the drug. Drugs with low pKa (3-7) are non ionised in the stomach and subsequently rapidly absorbed. On passage to the small intestine with comparatively increased pH, the rate of ionisation is changed and absorption subsequently slowed. The converse is true for drugs with higher pKa values.
   The stability of the drug through the pH range of the GI tract must also be considered.
(3) Physiological considerations include pH of the environment, the effect of gastric emptying time and variation of GI transit time. The pH is considered in (2). The effect of gastric emptying in the process of drug absorption is well documented.

Once an extended release dosage form passes beyond its principle absorption site in the GI tract, any further drug released may not contribute to therapy.

Factors which affect gastric emptying of the delivery system include fed or fasted state and the size of the delivery system.

EP 0235 718 discloses granules of drug that remain in the stomach. These granules are formed of a core comprising the active ingredient, a foaming layer of bicarbonate and organic acid coated on this core, and an expansive film coating this layer.

The present invention provides formulations for drugs, in particular hydrophilic drugs, which have a narrow window of absorption, limited predominantly to the stomach or the upper intestine as limited by their low pKa value (3-7) and/or their site of active transport absorption mechanism, but which require an extended release mechanism in order to (i) achieve a desired pharmacokinetic or bioavailability profile, (ii) overcome saturation of the active transport absorption mechanism and (iii) to overcome/reduce GI side effects due to the bolus release of the drug.

Furthermore, the present invention accomodates high dosage, highly soluble drugs in the formulation, allowing up to a 90% drug loading, thus minimising overall dosage unit weight/size and improving patient acceptability and compliance.

Furthermore, the formulation of the present invention has been designed in such a way as to allow optimum stability of the active component. It separates the active drug from acid and alkaline components in the formulation whilst allowing the formulation to maintain its novel behaviour.

The present invention relates to a floating extended release hydrophilic matrix formulation. The floating mechanism enables the delivery system to be maintained in the stomach for up to 4 hours, thus allowing optimum drug absorption as defined above, and maintaining an extended release of the drug to achieve desired pharmacokinetic and bioavailability profiles whilst reducing side effects. The claims are supported by pharmacoscintographic and pharmacokinetic studies to assess bioequivalence of a model active substance.

This invention can advantageously be applied to metformin.

Metformin hydrochloride has been successfully used for many years in the treatment of non insulin dependent diabetes.

Metformin is commercially available as 500 or 850 mg coated tablets. The usual posology is 500 mg every 8 hours or 850 mg every 12 hours, this posology is then adapted according to the biological results, to a maximum of 3 g daily in divided dose. At the beginning of the treatment, metformin may induce gastro-intestinal side effects such as diarrhoea and nausea.

Previous pharmacokinetic studies with oral metformin indicate that it has a narrow window of absorption at the upper part of the small intestine with a bioavailability of approximately 50%. This low bioavailability is thought to be due to a dose dependent saturation of receptors.

The present invention provides a new dosage form of metformin which will decrease the gastro-intestinal side effects at the beginning of the treatment and improves the bioavailability by sustaining the drug release in the stomach and optimising receptor uptake in the upper intestine.

### SUMMARY

The present invention relates to a floating extended release hydrophilic matrix formulation, in particular tablets, for the extended release of a drug, and to a process for their preparation.

The present invention relates to a tablet for extended release of a drug in the stomach, comprising granules of said drug in 2-hydroxypropylmethylcellulose, said granules being coated with a coating comprising a source of a carbon dioxide and said coated granules being blended with a pharmaceutically acceptable organic acid inducing the release of carbon dioxide and (a) tabletting aid(s).

The present invention relates in particular to tablets for the sustained release of any hydrophilic drugs with (i) a narrow absorption window limited to the stomach or upper GI and (ii) a low pKa value (3-7), whose bioavailability could be improved by sustained absorption in the upper GI, for example benzodiazepines (e.g. diazepam, chlordiazepoxide, nitrazepam), NSAIDs (e.g. indomethacin, naproxen, ibuprofen, fenoprofen), some antibacterials (e.g. sulphadiazine, isoniazid, flucloxacillin, ciprofloxacillin), metoprolol, minoxidil, hydralazine, methotrexate, aminophylline, chlorpromazine, fluphenazine, cimetidine, ranitidine, metformin, local anaesthesics (e.g. benzocaine), contrast media (e.g. barium sulphate) or any salts thereof.

The tablet according to the present invention may be obtained by a process comprising:
a) forming drug granules by wet granulation of a mixture of the hydrophilic drug and 2-hydroxypropylmethylcellulose ;
b) coating these granules with bicarbonate and binder ;
c) blending the coated granules with a tabletting aid and an organic acid, and,
d) tabletting the blend thus obtained into tablets, said 2-hydroxypropylmethylcellulose forming a hydrophilic matrix capable of retaining carbon dioxide which is formed when the tablet is administrated.

In general the concentration of the drug may be 10 to 90 % by weight of the tablet.

Thus the tablets of the present invention may contain :
10 to 90 % by weight of drug,
5 to 25 % by weight of 2-hydroxypropylmethylcellulose,
3 to 25 % by weight of bicarbonate,
1 to 10 % by weight of an organic acid,
0.5 to 30 % by weight of tabletting aid.

The 2-hydroxypropylmethylcellulose is a material which is able to form a hydrophilic matrix capable of retaining carbon dioxide formed when, in the stomach of the patient, the organic acid reacts with the bicarbonate.

Examples of appropriate grades of 2-hydroxypropylmethylcellulose are those having a methoxy range of 19 to 32 % by weight, a hydroxypropyl range of 4 to 12 % by weight and a viscosity of 15.10⁻³ Pa.s to 100 Pa.s in a 2 % aqueous solution at 20° C. The 2-hydroxypropylcellulose is preferably a polymer having a methoxy range of 19 to 24 % by weight, a hydroxypropyl range of 7 to 12 % by weight and a viscosity of about 100 Pa.s in a 2 % aqueous solution at 20° C. Such a grade is named HPMC 2208 under the USP specifications and is available under the name Methocel K100M.

Advantageously the mixture used for forming the granules comprises a granulating binder. This granulating binder is in particular a polyvinylpyrrolidone such as for example, a polyvinylpyrrolidone having a molecular weight of 45 000. The polyvinylpyrrolidone may be used in a proportion of 0.5 to 10 % with respect to the final tablet.

After the granulating step the granules may be sieved and dried. They are advantageously extruded and dried. The extrusion provides granules in the size range of 0.35 to 1.4 mm.

The granules are then mixed with the bicarbonate and a binder.

The source of carbon dioxide is in particular a bicarbonate of an alkali metal such as sodium or potassium carbonates or bicarbonates or sodium glycine carbonate. Sodium bicarbonate is the preferred source of carbon dioxide.

The binder used for coating with bicarbonate may be any binder usually used in order to increase the coating spreading efficiency of a powder on granules. This binder on the periphery of the granules will also facilitate the compression. This binder may be a polyvinylpyrrolidone such as PVP K30 (having a molecular weight of 45 000) or a 2-hydroxypropylmethylcellulose having a methoxy content of 28-30 % by weight, a hydroxypropyl content of 7-12 % by weight and a viscosity of 12-18.10⁻³ Pa.s, such as Methocel E15 LV.

This binder may be used in a proportion of 1 to 5 % by weight.

The coated granules are then blended with a tabletting aid and an organic acid.

The tabletting aid may be any aid usually used for making tablets. This aid is for example magnesium stearate.

Agents that induce the release of carbon dioxide are preferably pharmaceutically acceptable organic acids e.g. tartaric acid, malic acid, fumaric acid, adipic acid, succinic acid, ascorbic acid, maleic acid or preferably citric acid.

The tablets thus obtained may then be coated with a hydrophilic cellulose polymer and talc. The hydrophilic cellulose may be a 2-hydroxypropylmethylcellulose having a methoxy content of 28 to 30 % by weight, a hydroxypropyl content of 7 to 12 % and a viscosity of 12 to 18.10⁻³ pa.s as measured in a 2 % aqueous solution at 20° C.

For example the final coating of the tablet may comprise 0.5 to 5 % of said 2-hydroxypropylmethylcellulose such as Methocel E15 LV and 0.05 to 0.5 % of talc, said percentages being calculated with respect to the non-coated tablet.

### EXAMPLE 1

A tablet of metformin having the following composition has been prepared:

| Ingredients | mg/tablet | Weight percentage |
|---|---|---|
| Metformin hydrochloride | 500 | 62.42 |
| Methocel K100M | 127.5 | 15.9 |
| PVP K 30 | 36.9 | 4.6 |

| SPRAY | | |
|---|---|---|
| PVP K 30 | 13.25 | 1.6 |
| Sodium bicarbonate | 96.4 | 12 |

| Extragranular phase | | |
|---|---|---|
| Citric acid | 17.15 | 2.1 |
| Magnesium stearate* | 9.8 | 1.22 |

| | | |
|---|---|---|
| * A proportion of the magnesium stearate may be incorporated intragranularly if necessary. | | |

The tablets are prepared as follows :

### a) Granular stage

The metformin and Methocel K 100 M are blended in a suitable mixer.

The PVP K 30 solution is then added to the powder blend while granulating.

The wet powder is then extruded through a suitable screen, before being dried in a fluid bed dryer.

### b) Bicarbonate spraying stage

A bicarbonate/PVP solution is sprayed on the dry granules using a fluid bed coater.

### c) Compression stage

The dry sprayed granules are now blended with citric acid and with magnesium stearate in a suitable mixer.

The final blend is then compressed into tablets.

### EXAMPLE 2

A tablet of metformin having the following composition has been prepared:

| Ingredients | mg/tablet | Weight percentage |
|---|---|---|
| Metformin hydrochloride | 500 | 68.77 |
| Methocel K 15M | 50.56 | 6.94 |
| Methocel E 4M | 11.84 | 1.63 |
| PVP K 30 | 36.9 | 5.0 |

| SPRAY | | |
|---|---|---|
| Methocel E 15 LV | 13.25 | 1.8 |
| Sodium bicarbonate | 96.4 | 13.26 |

| Extragranular phase | | |
|---|---|---|
| Citric acid | 7.7 | 1.06 |
| Magnesium stearate* | 10.35 | 1.42 |

| | | |
|---|---|---|
| * A proportion of the magnesium stearate may be incorporated intragranularly if necessary. | | |

The tablets are prepared as follows :

### a) Granular stage

The Metformin, Methocel K 15 M and Methocel E 4 M100 are blended in a suitable mixer.

The PVP K 30 solution is then added to the powder blend while granulating.

The wet powder is then extruded through a suitable screen, before being dried in a fluid bed dryer.

### b) Bicarbonate spraying stage

A bicarbonate/Methocel E 15 LV solution is sprayed on the dry granules using a fluid bed coater.

### c) Compression stage

The dry sprayed granules are now blended with citric acid and with magnesium stearate in a suitable mixer.

The final blend is then compressed into tablets.

## Claims

1. A tablet for extended release of a drug in the stomach, comprising granules of said drug in a matrix of 2-hydroxypropylmethylcellulose, said granules being coated with a coating comprising a source of a carbon dioxide and said coated granules being blended with a pharmaceutically acceptable organic acid inducing the release of carbon dioxide and (a) tabletting aid(s).

2. A tablet as claimed in claim 1, wherein the drug is a hydrophilic drug.

3. A tablet as claimed in claim 1 or 2, wherein the organic acid is selected from the group consisting of tartaric acid, malic acid, fumaric acid, adipic acid, succinic acid, ascorbic acid, maleic acid and citric acid.

4. A tablet as claimed in claim 2 or 3, said tablet being obtained by :
a) forming drug granules by a wet granulation of a mixture of a hydrophilic drug and 2-hydroxypropylmethylcellulose ;
b) coating these granules with bicarbonate and a binder;
c) blending the coated granules with a tabletting aid and an organic acid, and
d) tabletting the blend thus obtained into tablets.

5. A tablet as claimed in claims 1, 2 or 3, wherein the drug is selected from benzodiazepines, NSAIDS, antibacterials, metoprolol, minoxidil, hydralazine, methotrexate, aminophylline, chlorpromazine, fluphenazine, cimetidine, ranitidine, metformine, local anaesthesics, contrast media and salts thereof.

6. A tablet as claimed in claims 2, 3 or 4, wherein the hydrophilic drug is a salt of metformin, e.g. metformin hydrochloride.

7. A tablet as claimed in anyone of claims 1 to 6 comprising :
10 to 90% by weight of drug,
5 to 25% by weight of 2-hydroxypropylmethylcellulose,
3 to 25% by weight of bicarbonate,
1 to 10% by weight of an organic acid,
0.5 to 30% by weight of a tabletting aid.

8. A tablet as claimed in anyone of claims 1 to 7 comprising polyvinylpyrrolidone as binder for the coating with bicarbonate.

9. A tablet as claimed in claim 8 comprising 1 to 5% of polyvinylpyrrolidone.

10. A tablet as claimed in anyone of claims 1-9 wherein the 2-hydroxypropylmethylcellulose has a methoxy range of 19 to 32% by weight, a hydroxypropyl range of 4 to 12% by weight and a viscosity of 15.10⁻³ Pa.s to 100 Pa.s in a 2% aqueous solution at 20° C.

11. A tablet as claimed in claim 10, wherein the 2-hydroxypropylmethylcellulose has a methoxy range of 19 to 24% by weight, a hydroxypropyl range of 7 to 12% by weight and a viscosity of about 100 Pa.s in a 2% aqueous solution at 20° C.

12. A tablet as claimed in anyone of claims 1 to 11 comprising a coating of a hydrophilic cellulose polymer and talc.

13. A process for preparing a tablet as claimed in claim 4, comprising:
a) forming hydrophilic drug granules by a wet granulation of a mixture of the hydrophilic drug and 2-hydroxypropylmethylcellulose ;
b) coating these granules with bicarbonate and a binder;
c) blending the coated granules with a tabletting aid and an organic acid, and
d) tabletting the blend thus obtained into tablets.

## Patentansprüche

1. Tablette zum verlängerten Freisetzen eines Medikaments im Magen, umfassend Körnchen dieses Medikaments in einer Matrix aus 2-Hydroxypropylmethylcellulose, wobei diese Körnchen mit einer Beschichtung, umfassend eine Kohlendioxidquelle, beschichtet sind und diese beschichteten Körnchen mit einer pharmazeutisch verträglichen organischen Säure, die das Freisetzen von Kohlendioxid induziert, und einem Tablettierungshilfsmittel oder Tablettierungshilfsmitteln, vermischt sind.

2. Tablette nach Anspruch 1, worin das Medikament ein hydrophiles Medikament ist.

3. Tablette nach Anspruch 1 oder 2, worin die organische Säure ausgewählt ist aus der Gruppe, bestehend aus Weinsäure, Äpfelsäure, Fumarsäure, Adipinsäure, Succinsäure, Ascorbinsäure, Maleinsäure und Zitronensäure.

4. Tablette nach Anspruch 2 oder 3, erhältlich durch
a) Bilden von Medikamentenkörnchen über Nassgranulierung eines Gemischs eines hydrophilen Medikaments und 2-Hydroxypropylmethylcellulose,
b) Beschichten dieser Körnchen mit Bicarbonat und einem Bindemittel,
c) Vermischen der beschichteten Körnchen mit einer Tablettierungshilfe und einer organischen Säure, und
d) Tablettierung des so erhaltenen Gemischs in Tabletten.

5. Tablette nach einem der Ansprüche 1, 2 oder 3, worin das Medikament ausgewählt ist aus Benzodiazepin, NSAIDS, Bakteriziden, Metoprolol, Minoxidil, Hydralazin, Methotrexat, Aminophyllin, Chlorpromazin, Fluphenazin, Cimetidin, Ranitidin, Metformin, Lokalanaesthetica, Kontrastmittel und deren Salzen.

6. Tablette nach einem der Ansprüche 2, 3 oder 4, worin das hydrophile Medikament ein Salz aus Metformin, z. B. Metforminhydrochlorid ist.

7. Tablette nach einem der Ansprüche 1 bis 6, enthaltend
10 bis 90 Gew.% Medikament
5 bis 25 Gew.% 2-Hydroxypropylmethylcellulose
3 bis 25 Gew.% Bicarbonat
1 bis 10 Gew.% einer organischen Säure
0,5 bis 30 Gew.% einer Tablettierungshilfe.

8. Tablette nach einem der Ansprüche 1 bis 7, enthaltend Polyvinylpyrrolidon als Bindemittel zum Beschichten mit Bicarbonat.

9. Tablette nach Anspruch 8, enthaltend 1 bis 5 % Polyvinylpyrrolidon.

10. Tablette nach einem der Ansprüche 1 bis 9, worin die 2-Hydroxypropymethylcellulose einen Gehalt an Methoxy von 19 bis 32 Gew.%, einen Gehalt an Hydroxypropyl von 4 bis 12 Gew.% und eine Viskosität von 15 x 10⁻³ Pa.s bis 100 Pa.s in einer 2 %-igen wässrigen Lösung bei 20 °C hat.

11. Tablette nach Anspruch 10, worin die 2-Hydroxypropylmethylcellulose einen Gehalt an Methoxy von 19 bis 24 Gew.%, einen Gehalt an Hydroxypropyl von 7 bis 12 Gew.% und eine Viskosität von etwa 100 Pa.s in einer 2 %-igen wässrigen Lösung bei 20 °C hat.

12. Tablette nach einem der Ansprüche 1 bis 11, enthaltend eine Beschichtung aus einem hydrophilen Cellulosepolymer und Talk.

13. Verfahren zur Herstellung von Tabletten nach Anspruch 4, umfassend
a) Bilden hydrophiler Medikamentenkörnchen über Nassgranulierung eines Gemischs aus hydrophilem Medikament und 2-Hydroxypropylmethylcellulose,
b) Beschichten dieser Körnchen mit Bicarbonat und einem Bindemittel,
c) Vermischen der beschichteten Körnchen mit einer Tablettierungshilfe und einer organischen Säure,
d) Tablettierung des so erhaltenen Gemischs in Tabletten.

## Revendications

1. Comprimé pour libération prolongée d'un médicament dans l'estomac, comprenant des granules dudit médicament dans une matrice de 2-hydroxypropylméthylcellulose, lesdits granules étant recouverts d'un enrobage comprenant une source d'un dioxyde de carbone et lesdits granules enrobés étant mélangés avec un acide organique pharmaceutiquement acceptable induisant la libération du dioxyde de carbone et d'une ou plusieurs substance(s) contribuant à la formation des comprimés.

2. Comprimé selon la revendication 1, dans lequel le médicament est un médicament hydrophile.

3. Comprimé selon la revendication 1 ou 2, dans lequel l'acide organique est choisi dans le groupe consistant en acide tartrique, acide malique, acide fumarique, acide adipique, acide succinique, acide ascorbique, acide maléique et acide citrique.

4. Comprimé selon la revendication 2 ou 3, lequel comprimé étant obtenu par les étapes suivantes : a) formation de granules de médicament par une granulation par voie humide d'un mélange d'une substance hydrophile et de 2-hydroxypropylméthylcellulose ; b) enrobage de ces granules avec du bicarbonate et un liant ; c) mélange des granules enrobés avec une substance contribuant à la formation des comprimés et un acide organique, et d) formation du mélange ainsi obtenu en comprimés.

5. Comprimé selon la revendication 1, 2 ou 3, dans lequel le médicament est choisi dans le groupe composé de benzodiazépines, NSAIDS, antibactériens, métoprolol, minoxidil, hydralazine, méthotrexate, aminophylline, chlorpromazine, fluphenazine, cimétidine, ranitidine, metformine, anesthésiques locaux, produit de contraste et sels de ceux-ci.

6. Comprimé selon les revendications 2,3 ou 4, dans lequel la substance hydrophile est un sel de metformine, par ex. du chlorhydrate de metformine.

7. Comprimé selon l'une quelconque des revendications 1 à 6 comprenant: 10 à 90 % en poids de médicament, 5 à 25 % en poids de 2-hydroxypropylméthylcellulose, 3 à 25 % en poids de bicarbonate, 1 à 10 % en poids d'acide organique, 0,5 à 30 % en poids d'une substance contribuant à la formation des comprimés.

8. Comprimé selon l'une quelconque des revendications 1 à 7 comprenant de la polyvinylpyrrolidone comme liant pour l'enrobage avec du bicarbonate.

9. Comprimé selon la revendication 8 comprenant 1 à 5 % de polyvinylpyrrolidone.

10. Comprimé selon l'une quelconque des revendications 1 à 9 dans lequel la 2-hydroxypropylméthylcellulose a une teneur en méthoxy de 19 à 32 % en poids, une teneur en hydroxypropyl de 4 à 12 % en poids et une viscosité de 15.10⁻³ Pa.s à 100 Pa.s dans une solution aqueuse 2 % à 20°C.

11. Comprimé selon la revendication 10, dans lequel la 2-hydroxypropylméthylcellulose a une teneur en méthoxy de 19 à 24 % en poids, une teneur en hydroxypropyl de 7 à 12 % en poids et une viscosité d'environ 100 Pa.s dans une solution aqueuse 2 % à 20°C.

12. Comprimé selon l'une quelconque des revendications 1 à 11 comprenant un enrobage d'un polymère de cellulose hydrophile et de talc.

13. Procédé pour préparer un comprimé selon la revendication 4, comprenant les étapes suivantes :
a) formation de granules de médicament par une granulation par voie humide d'un mélange d'un médicament hydrophile et de 2-hydroxypropylméthylcellulose ;
b) enrobage de ces granules avec du bicarbonate et un liant ;
c) mélange des granules enrobés avec une substance contribuant à la formation des comprimés et un acide organique, et
d) formation du mélange ainsi obtenu en comprimés.
